# DEMANDE DE BREVET EUROPEEN

(11) **EP 2 272 560 A1**
(43) Date de publication de la demande: **12.01.2011**
(21) Numéro de dépôt: 09008907.9
(22) Date de dépôt: 08.07.2009
(51) Int. Cl.: A61N 1/05, A61N 1/362, A61N 1/39

(54) **Dispositif integre de cardioversion et stimulation atriales transoesophagiennes**

(71) Demandeur: Prothia S.A.R.L., 75020 Paris (FR)
(72) Inventeur: Spear, Brian Hartwell, 75013 Paris (FR)

(57) **Abrégé**

L'invention concerne un système oesophagien capable de recueillir l'activité électrocardiographique supraventriculaire et de générer sans intervention chirurgicale ni implantation des chocs ou des stimuli thérapeutiques appropriés. Il s'agit d'un système composé d'une sonde munie d'électrodes, d'un générateur de chocs, d'un générateur de stimuli, d'une électronique de recueil des traitements des signaux électrocardiographiques ainsi que des connecteurs des contrôles des affichages et des alimentations adaptés à l'application.

Le dispositif est caractérisé selon l'invention par le fait qu'un seul et même générateur connecté à une seule et même sonde accomplit les fonctions de cardio-version et de stimulation atriales transoesophagiennes, les électrodes de la sonde ont une forme et une architecture assurant à la fois un bon recueil, une stimulation efficace de faible énergie et une cardio-version efficace de faible énergie.

Les électrodes (2) sont constituées de deux ou plusieurs anneaux (1) séparés pour que la sonde reste souple mais connectée au plan électrique pour créer une grande masse conductrice

## Description

La présente invention concerne un système médical non chirurgical, temporaire et non- implantable, pour appliquer des énergies électriques à une partie du corps par voie oesophagienne.

Des millions d'adultes de part le monde souffrent de problèmes cardiaques appelés rythmologiques. Il s'agit de personnes dont le coeur ne se contracte pas de façon régulière et efficace ; chez ces malades, le muscle cardiaque, au lieu de se contracter de façon régulière et organisée, se comporte différemment. C'est-à-dire, ces coeurs pathologiques ont des parties qui se contractent trop lentement, ou se contractent tellement vite que les différentes chambres n'ont pas le temps de se remplir et se vider correctement, ou qui tremblent et ne se contractent pas. Ces rythmes portent des noms selon leur nature, tels : bradycardie, tachycardie, flutter, fibrillation. Aussi, on précise si la partie du corps concernée est la partie critique au plan de l'efficacité du coeur à savoir ventriculaire, ou concerne les chambres supérieures du coeur nommées supraventriculaires ou atriales.

Différentes thérapies s'appliquent à des patients dysrythmiques dont des traitements électriques. Plus précisément, on administre des chocs appelés chocs de cardio-version qui ont pour but de réorganiser une activité électrique du coeur plutôt anarchiques (fibrillation ou flutter); et de façon similaire, on applique des stimuli pour augmenter la fréquence cardiaque dans un premier temps afin de la ramener à un niveau acceptable dans un deuxième temps - technique appelée « overdrive ».

Deux voies principales sont utilisées pour apporter le courant au muscle cardiaque : la voie endocavitaire où une sonde est placée dans la chambre concernée, la voie trans-cutanée ou transthoracique où des électrodes sont placées sur la peau. La voie endocavitaire nécessite une intervention sanglante et entraîne des risques liés au placement d'une sonde dans les vaisseaux et dans le coeur. La voie trans-cutanée nécessite l'utilisation de très fortes énergies pour que la quantité de courant requise arrive à destination c'est-à-dire au muscle cardiaque. Pour que ces énergies soient appliquées à bon escient, les médecins utilisent des technologies qui recensent l'activité électrique du coeur pour que l'application de chocs et de stimuli soit synchronisée à certains évènements électriques du coeur. Ces différents évènements électriques se désignent généralement par leur place et leur morphologie affichées ou enregistrées dans l'électrocardiogramme (ou l'ECG).

La voie oesophagienne représente une voie alternative peu utilisée à ce jour. Aussi en 1986 Monsieur le Docteur Jean Yves Artigou a publié (La lettre du cardiologue N°63 - mai 1986) ses résultats satisfaisants de stimulation atriale trans-oesophagienne réalisée avec une sonde conçue par l'inventeur. Cette sonde s'utilise encore à ce jour pour la stimulation, mais elle ne convient pas tout à fait aux applications de choc visées par la présente invention. D'autres dispositifs brevetés s'intéressent à la voie oesophagienne. Un brevet de William Metzger (1990) reprend plusieurs aspects de la construction développée par l'auteur de la présente invention et décrite dans l'article d'Artigou cité plus haut. D'autres dispositifs oesophagiens sont de diamètre trop important pour une application temporaire et sans anesthésie et ne peuvent convenir aux introductions par le nez. D'autres inventions notamment celle de Friedman (2006) s'intéressent à des systèmes implantables de stimulation et de choc par voie oesophagienne.

Or pour faciliter l'application des énergies thérapeutiques par voie oesophagienne et ceci en dehors d'indications justifiant d'un dispositif implantable, certains cliniciens pour certains patients pourraient préférer l'introduction de la sonde par le nez, c'est pourquoi notre dispositif est de relativement petit diamètre, environ 3 mm de diamètre ou moins.

Au plan anatomique, l'oesophage est à quelques centimètres de la partie supraventriculaire du coeur. La voie oesophagienne a de nombreux avantages pour l'application d'énergie à l'oreillette, un nom qui désigne la partie atriale du coeur : l'absence de nécessité d'anesthésie générale, le fait de ne pas avoir besoin de recourir à des techniques sanglantes et le faible niveau d'énergie nécessaire pour stimuler ou choquer le coeur. L'absence de matériel bien adapté à appliquer les énergies électriques par voie oesophagienne et sans chirurgie, figure parmi les raisons pour lesquelles la voie oesophagienne est peu utilisée à ce jour.

La présente invention se propose d'apporter une solution simple et peu coûteuse pour envoyer des chocs et des stimuli destinés à l'oreillette. A cet effet, l'invention concerne un système oesophagien capable de recueillir l'activité électrocardiographique supraventriculaire et de générer des chocs ou des stimuli thérapeutiques appropriés. Il s'agit d'un système composé d'une sonde munie d'électrodes, d'un générateur de chocs, d'un générateur de stimuli, d'une électronique de recueil des traitements des signaux électrocardiographiques ainsi que des connecteurs de contrôle des affichages et des alimentations adaptés à l'application temporaire, non-implantée, non chirurgicale

Le dispositif inclut un seul et même générateur connecté à une seule et même sonde pour fournir les fonctions de cardio-version et de stimulation atriales transoesophagiennes.

La sonde a une forme et une architecture assurant à la fois un bon recueil, une stimulation efficace de faible énergie et une cardio-version efficace de faible énergie. La construction de la sonde est différente de celle des sondes connues à ce jour.

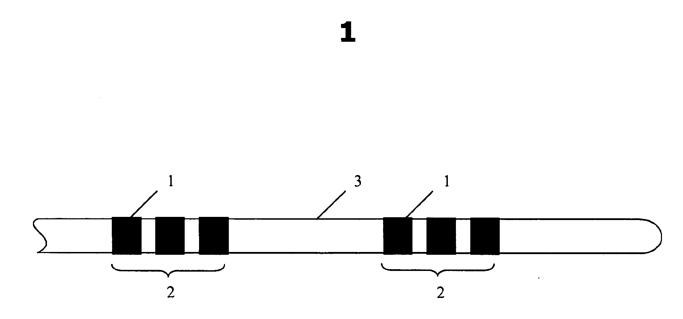

La figure 1 représente la partie distale de la sonde avec ses électrodes. Selon un aspect avantageux de l'invention, bien que non limitatif, les anneaux (1) des électrodes (2) ont un diamètre plus grand que celui du corps (3) de la sonde. Les électrodes (2) sont constituées de deux ou plusieurs anneaux (1) séparés pour que la sonde reste souple mais connectée au plan électrique pour créer une grande masse conductrice.

L'espace entre les électrodes multi annulaires ou multi articulaires, est égal ou supérieur à deux centimètres. Selon un aspect avantageux de l'invention, bien que non limitatif, une partie souple de la sonde est distale à l'électrode distale de la sonde, de telle sorte qu'une partie souple sans électrode est introduite en premier dans le nez ou la bouche du patient. De préférence, la sonde comporte deux électrodes ou plus, et chaque électrode consiste en deux éléments ou plus.

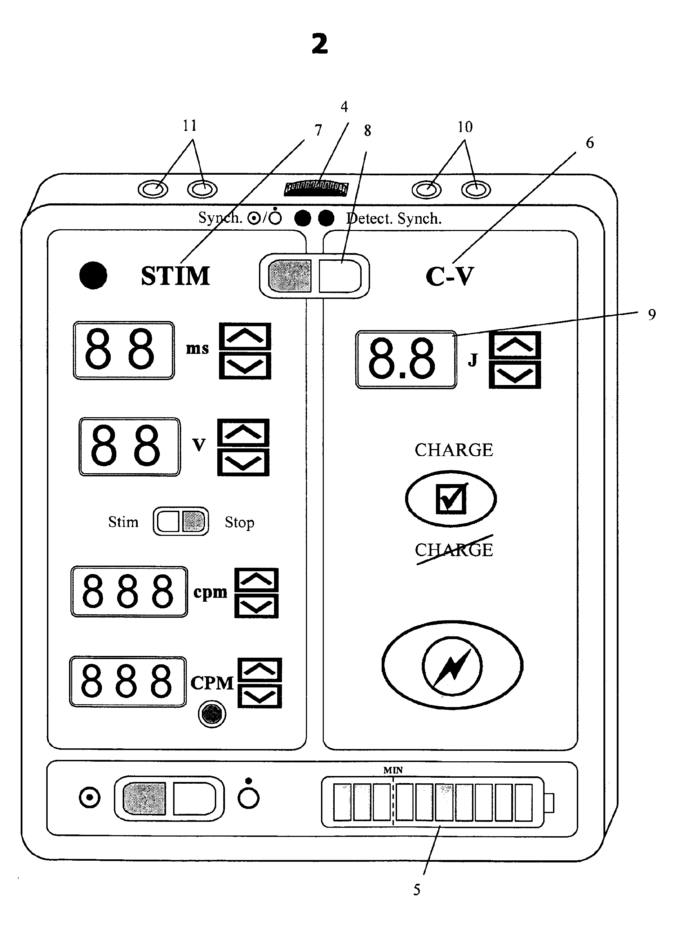

La figure 2 représente une version avantageuse du générateur bien que non limitative où les fonctions de stimulation et de cardio-version se trouvent réunies. Le choix de sensibilité (4) est commun aux fonctions de cardio-version et de stimulation. L'alimentation, représentée selon un aspect avantageux mais non limitatif, par un indicateur de batterie (5), est commune aux fonctions de cardio-version (6) et de stimulation (7). Selon un aspect avantageux de l'invention, bien que non limitatif, la définition du niveau d'énergie (8) est d'un dixième de Joule pour des niveaux inférieurs à 10 Joules ; cette définition par dixième dans le domaine de la cardio-version est une innovation liée à la fois à la conception du dispositif objet du présent brevet et aux possibilités offertes pour réaliser des cardio-versions supraventriculaires par voie oesophagienne.

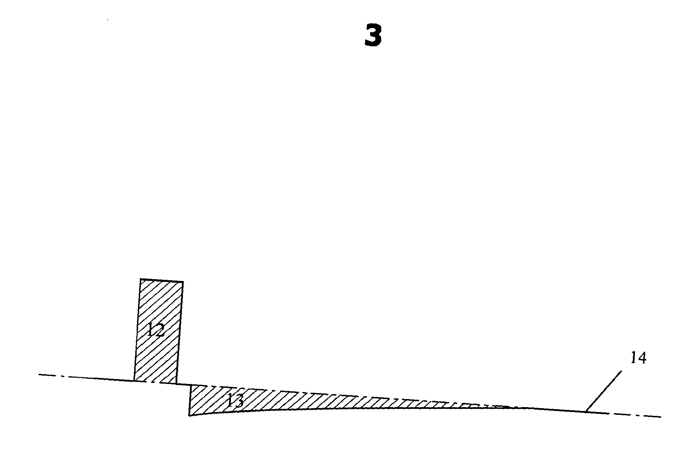

La figure 3 représente une version avantageuse mais non limitative du protocole de stimulation avec une représentation du courant de dépolarisation des électrodes. Une des difficultés rencontrées dans la stimulation atriale transoesophagienne est la création d'une polarisation liée à la tension nécessaire à entraîner une contraction de l'oreillette par l'oesophage. La polarisation du coeur se réalise par un stimulus représenté sur la figure 3 par la partie (12). Après la stimulation, la repolarisation se fait par un courant en sens contraire, soit à polarité inversé. Ce courant de repolarisation de faible amplitude et négatif est représenté par l'aire de l'onde (13) sur la figure 3. Selon un aspect avantageux mais non limitatif de l'invention, le stimulus est positif, c'est à dire au-dessus de la ligne électrisée (14) et l'impulsion de repolarisation(13) est négative, c'est à dire en dessous de cette même ligne. Le stimulus (12) et l'impulsion de repolarisation (13) sont de charge égale.

## Revendications

1. Dispositif intégré de cardio-version et stimulation atriales transoesophagiennes à visée temporaire et non-implantable, composé d'une sonde munie d'électrodes, d'un générateur de chocs, d'un générateur externe et portable de stimuli, d'une électronique de recueil des traitements des signaux électrocardiographiques ainsi que de connecteurs de contrôle des affichages et des alimentations adaptés à l'application, **caractérisé par le fait qu'**il comprend un seul et même générateur connecté à une seule et même sonde destinés à accomplir les fonctions de cardio-version et de stimulation atriales transoesophagiennes, que l'électronique de recueil et de traitement électrocardiographiques est commune aux fonctions de stimulation et de cardio-version, que l'alimentation des fonctions de cardio-version et de stimulation est également partagée et que les électrodes de la sonde ont une forme et une architecture assurant à la fois un bon recueil, une stimulation efficace de faible énergie et une cardio-version efficace de faible énergie.

2. Dispositif selon la revendication 1 **caractérisé en ce que** la sonde (Figure 1) comporte deux électrodes (2) ou plus, et chaque électrode consiste en deux éléments (1) ou plus.

3. Dispositif selon la revendication 1 **caractérisé en ce que** les électrodes (2) de la sonde sont composées de plusieurs anneaux (1) ou articulations séparées (1) pour maintenir la souplesse de la sonde en augmentant la masse conductrice effective et pour assurer un plus grand contact entre les électrodes (2) et la paroi de l'oesophage.

4. Dispositif selon la revendication 1 **caractérisé en ce que** les anneaux (1) des électrodes (2) ont un diamètre plus grand que celui du corps (3) de la sonde.

5. Dispositif selon la revendication 1 **caractérisé en ce qu'**une partie souple de la sonde est distale à l'électrode distale de la sonde, de telle sorte qu'une partie souple sans électrode est introduite en premier dans le nez ou la bouche du patient.

6. Dispositif selon la revendication 1 **caractérisé en ce que** le diamètre de la sonde est suffisamment petit pour passer la sonde par une narine d'adulte (environ 3 mm).

7. Dispositif selon la revendication 1 **caractérisé en ce que** la sonde accepte un mandrin pour faciliter la manipulation de la sonde et sa rigidification une fois passée dans l'oesophage.

8. Dispositif selon la revendication 1 **caractérisé en ce que** la charge des stimulus « overdrive » (12) est égale et de polarité inverse à la charge de l'impulsion de repolarisation (13).

9. Dispositif selon la revendication 1 **caractérisé en ce que** la durée des stimuli « overdrive » (12) est plus courte que la durée de l'impulsion de repolarisation (13).
